# EUROPEAN PATENT APPLICATION

(11) **EP 1 055 366 A1**
(43) Date of publication of application: **29.11.2000**
(21) Application number: 99110169.2
(22) Date of filing: 26.05.1999
(51) Int. Cl.: A01K 67/027, C12N 5/06, C07K 14/82, C07K 14/47, G01N 33/50

(54) **USE OF TRANSGENIC MICE LACKING JUN-B EXPRESSION IN THE MYELOID LINEAGE**

(71) Applicant: Boehringer Ingelheim International GmbH, 55218 Ingelheim am Rhein (DE)
(72) Inventor: Passegue, Emmanuelle Dr., 1040 Wien (AT); Jochum, Wolfram Dr., 2514 Traiskirchen (AT); Wagner, Erwin Prof. Dr., 1030 Wien (AT)
(74) Representative: Laudien, Dieter, Dr.

(57) **Abstract**

The use of a transgenic animal lacking JunB expression in the myeloid lineage as a model for human chronic myeloid leukemia

## Description

The present invention relates to the field of myeloid leukemias.

Myelopoiesis, the regulated process which produces granulocytes and monocytes, involves mechanisms such as cell differentiation, progenitor cell proliferation, survival and apoptosis. A number of transcriptional regulators critical to myeloid differentiation have been identified (reviewed in Tenen et al., 1997), however, less is known about transcription factors which modulate cell numbers in the myeloid lineage by controlling proliferation and cell death.

AP-1 (activating protein-1) is a dimeric transcription factor composed of Jun, Fos or ATF (activating transcription factor) family members that binds to a common DNA site, the AP-1 binding site, and converts extracellular signals into changes in the transcription of many cellular and viral genes (reviewed in Angel and Karin, 1991). AP-1 activity is modulated by various signals including growth factors, cytokines, tumor promoters, carcinogens and specific oncogenes. AP-1 has been implicated in a number of biological processes such as cell proliferation, cell differentiation and apoptosis. However, analysis of AP-1 functions *in vivo* and in tissue culture cells has shown that different AP-1 members regulate different target genes and thus execute distinct biological functions in a cell-type specific fashion.

All the Jun proteins (c-Jun, JunB and JunD) are similar with respect to their primary structure and their DNA-binding specificity. However, JunB, due to a small number of amino acid changes in its basic-leucine zipper region exhibits a 10-fold weaker DNA-binding activity and a decreased homodimerization property compared to c-Jun. Transfection studies have suggested that JunB is a repressor of AP-1-mediated transactivation since JunB efficiently repressed c-Jun mediated transactivation and transformation most likely due to the preferential formation of inactive c-Jun/JunB dimers (Chiu et al., 1989; Deng and Karin, 1993). However, *in vitro* and *in vivo* data have demonstrated that JunB can also be a strong transactivator depending on both the interacting partner and the promoter context (Chiu et al., 1989; Li et al., 1999). It was recently shown that fetuses lacking *jun*B die around day 9.5 of embryonic development due to defective feto-maternal interactions (Schorpp-Kistner et al., 1999). In contrast, transgenic mice constitutively expressing the mouse *jun*B gene under the control of the human ubiquitin C promoter (Ubi-*jun*B) in many different tissues have no obvious phenotype (Schorpp et al., 1996). Schorpp-Kistner et al. (1999) also showed that the embryonic lethality of *jun*B mutant embryos could be rescued by the Ubi-*jun*B transgene indicating that the lack of *jun*B was solely responsible for the lethal phenotype in the embryo.

JunB has been implicated in the control of proliferation and differentiation of various cell types including myeloid cells (Schlingensiepen et al. 1993; Lord et al., 1990). JunB is constitutively expressed in human mature granulocytes (Mollinedo et al., 1991). Furthermore, JunB expression is strongly induced following terminal differentiation of bone marrow myeloid precursors and established myeloid cell lines such as HL-60, U937 and M1 (Lord et al., 1990; Mollinedo and Naranjo, 1991; Lord et al., 1993). It was thus postulated that JunB may play an important role in promoting myeloid differentiation and it was speculated that genetic alterations affecting JunB expression may contribute to leukemogenesis. However, the role of *jun*B has never been established.

Human chronic myeloid leukemia (CML) is a malignant clonal disorder of hematopoietic stem cells characterized by increased proliferation of the myeloid lineage (reviewed in Deininger, 1998; Sawyers, 1999). CML accounts for about 25% of adult leukemias. The principal clinical features include elevated white cell counts in the peripheral blood, hepato-splenomegaly, and myeloid hyperplasia of the bone marrow. The natural course of the disease is progressive. The initial chronic phase is followed by a rapidly fatal blast crisis within three to five years. In 90 to 95% of CML patients, the Philadelphia chromosome is present, a shortened chromosome 22 that arises from a reciprocal translocation, t(9;22) (q34;q11) and serves as a cytogenetic hallmark of the disease. The treatment of CML is largely palliative, since only a small percentage of patients can undergo potentially curative therapeutical procedures such as allogeneic bone marrow or stem cell transplantation. During the chronic phase of CML, cytoreductive therapy using oral chemotherapeutic drugs such as busulfan and hydroxyurea is able to induce haematologic remissions and to maintain the neutrophil count in the normal range to avoid thrombotic complications. Alternatively, interferon alpha can induce haematologic and cytogenetic remissions in patients with chronic phase CML. In contrast, the blastic phase of CML is refractory to most chemotherapeutic drug regimens, although short-term remissions in a small percentage of patients have been obtained. The poor prognosis of blast crisis patients clearly indicates an urgent need for more effective modes of treatment. However, advances along that line require a better understanding of the molecular events which cause or accompany the transition to blast crisis stage of CML.

A number of experimental approaches have been taken to generate animal models for human CML in order to identify mechanisms contributing to leukemogenesis and to ultimately design effective CML therapies interfering with these mechanisms. These approaches can be grouped in three categories:

### 1. Transfer of human myeloid leukemia cells into SCID mice

Established growth factor-independent human myeloid leukemia cell lines (K562, U937) or peripheral blood/bone marrow cells derived from patients with chronic myeloid leukemia in blast crisis were injected into severe immunodeficient (SCID) mice to induce a CML-like disease (Sawyers 1992, Cesano 1992).

### 2. Transduction of genes into hematopoietic stem cells using retroviral vectors

Using retroviral vectors, wild-type bone marrow cells were transduced with genes encoding hematopoietic growth factors, oncogenes and mutated tumor suppressor genes such as G-CSF (Chang et al., 1989), GM-CSF (Johnson et al., 1989), IL-3 (Chang et al., 1989; Wong et al., 1989), IL-6 (Hawley et al., 1992), PDGF-B (Yan et al., 1994), v-*src* (Keller and Wagner, 1989), Bcr-abl (Daley et al., 1990; Elefanty et al., 1990; Kelliher et al., 1990), v-*myc* (Bonham et al., 1992), TEL/JAK2 (Schwaller et al., 1998), N-*ras* (MacKanzie et al., 1999) and mutant p53 (Shounan et al., 1997). The genetically modified bone marrow was introduced into lethally irradiated wild-type recipient mice, which developed a myeloproliferative syndrome after various periods of time.

### 3. Genetically engineered mice

Transgenic mice expressing a CML-associated Bcr-Abl protein under the control of the metallothionein-1 promoter develop acute leukemia at birth (Heisterkamp et al., 1990). Mice lacking ICSBP (Holtschke et al., 1996), Ship (Helgason et al., 1998) or Nf-1 (Largaespada et al., 1996) in the hematopoietic system develop a myeloproliferative syndrome, which in the case of ICSBP mutant and Nf-1 mutant mice can progress to a blast crisis.

It was in object of the invention to provide an alternative animal model for human CML which is particularly suitable to study the transition from chronic phase to blast crisis and to elucidate the role of JunB in tumorigenesis of hematopoetic cells in order to harness these findings for the therapy of leukemias.

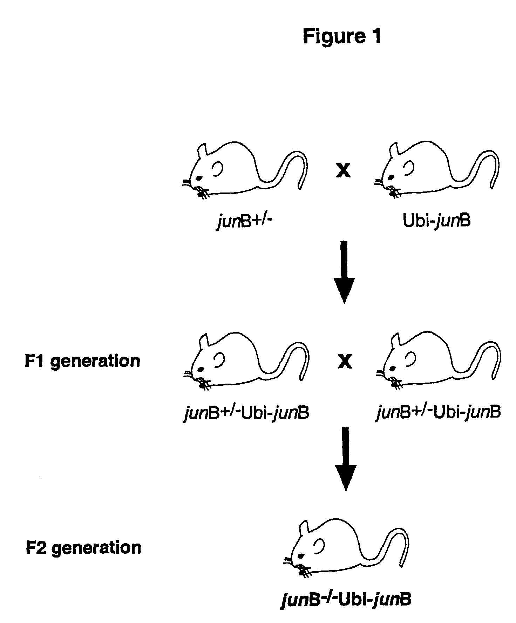

The role of *jun*B in the hematopoietic system was studied using *jun*B^{-/-}Ubi-*jun*B transgenic mice. These mice were obtained according to the method described by Schorpp-Kistner et al. (1999). In brief, Ubi-*jun*B transgenic mice were crossed to *jun*B mutant heterozygous mice and the transgenic *jun*B mutant heterozygous progenies were intercrossed to generate mice homozygous for the inactivated *jun*B allele that carry the Ubi-*jun*B transgene (Fig. 1). Such *jun*B^{-/-}Ubi-*jun*B mice were obtained in the expected Mendelian ratio (Table 1).

It was surprisingly found in the experiments of the invention that *jun*B^{-/-}Ubi-*jun*B mice develop a myeloproliferative disease due to lack of JunB expression in the myeloid lineage.

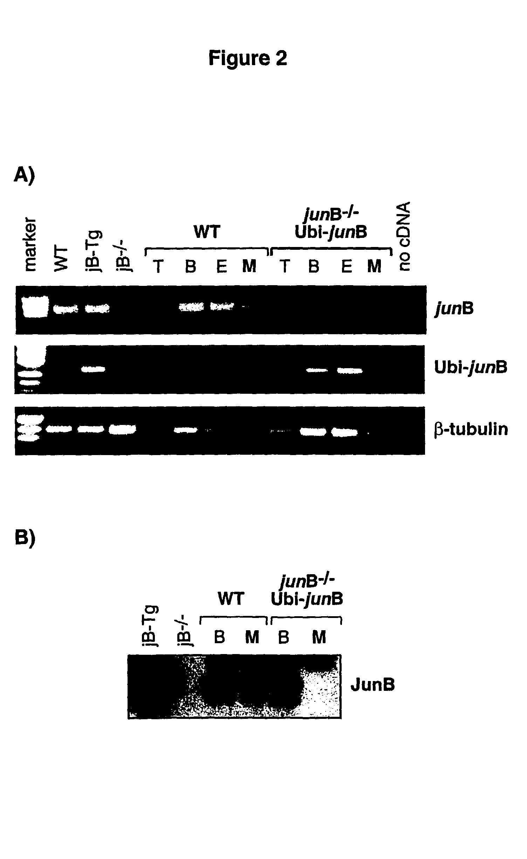

In a first set of experiments, JunB expression was analyzed, at the mRNA and protein level, in various hematopoietic lineages derived from *jun*B^{-/-}Ubi-*jun*B mice. It was found that all hematopoetic lineages express JunB except for the myeloid lineage (Fig. 2).

All *jun*B^{-/-}Ubi-*jun*B mice develop a myeloproliferative disease with complete penetrance by 11 months of age. The myeloproliferative disease was characterized by a progressive and selective enlargement of the granulopoietic compartment in hematopoietic organs (Fig. 3 and Table 2). In a certain proportion of the *jun*B^{-/-}Ubi-*jun*B mice the disease progresses to a fatal blast crisis. Bone marrow transplantation experiments demonstrate that the myeloproliferative disease of *jun*B^{-/-}Ubi-*jun*B mice is due to a cell autonomous defect within the myeloid lineage (Fig. 4). It was finally shown that myeloid cells lacking JunB expression display increased proliferation *in vivo* (Fig. 5).

The experiments of the present invention have thus shown that JunB acts as a negative regulator of myelopoiesis by controlling the proliferation of myeloid progenitors without interfering with myeloid maturation.

The myeloproliferative disorder observed in *jun*B^{-/-}Ubi-*jun*B mice surprisingly recapitulates essential features of human CML. Absence of JunB expression in the myeloid lineage leads to increased myeloid proliferation and consecutive myeloid hyperplasia. Consistent with the model of multistage tumorigenesis, a certain proportion of *jun*B^{-/-}Ubi-*jun*B mice develop blast crisis in the course of the myeloproliferative disease indicating that hyperproliferation predisposes to additional genetic alterations that eventually lead to blastic transformation. The similarities of the myeloproliferative disease in *jun*B^{-/-}Ubi-*jun*B mice with human CML indicates that these mice are a very efficient tool to identify genes that contribute to leukemogenesis, especially blastic transformation.

In a first aspect, the present invention relates to the use of transgenic animals lacking JunB expression in the myeloid lineage as a model for human CML.

Since the technology of gene manipulation is best developed for mice, in a preferred embodiment, the animal is a mouse. However, other mammals like rats, monkeys, pigs, etc. may be used applying methods that allow for equivalent gene manipulation leading to the corresponding phenotype.

Several ways of generating mice that lack *jun*B in the myeloid lineage are available.

According to a first method, the mice may be obtained by transgene complementation of a conventional *jun*B knock-out applying a *jun*B transgene in which *jun*B is under the control of a strong and constitutive promoter that leads to expression during early embryonic development. An example is the human ubiquitin C promoter (Schorpp et al., 1996), which was used in the experiments of the present invention.

In an alternative method, the mice may be generated by conditionally inactivating *jun*B in the myeloid lineage by established genetic approaches. Examples for such a conditional inactivation approach are the well-established Cre/loxP system (Araki et al., 1995) and the Flp/FRT system (Dymecki, 1996). In the Cre/loxP system, floxed *jun*B knock-in mice are generated by standard ES-cell technology as described by Capecchi (1989), by injecting blastocysts with ES-cells in which the endogenous *jun*B gene was replaced by a floxed *jun*B allele (entire *jun*B coding sequence flanked by two loxP sites). Floxed *jun*B mice are then crossed to transgenic mice expressing the Cre recombinase in the myeloid lineage, preferably in myeloid progenitor cells. The promoter elements of a number of myeloid-specific genes have been demonstrated to target transgene expression to myeloid cells such as the promoters of the human HMRP8 gene (Lagasse and Weissmann, 1994; Brown et al., 1997) and the human cathepsin G gene (Grisolano et al., 1997). However, every promoter element which directs CRE recombinase activity to the myeloid lineage is suitable for this approach. A similar strategy can be applied for the Flp/FRT system by flanking *jun*B by FRT sites and by expressing the Flp recombinase under the control of a myeloid specific promoter.

Another method for obtaining mice with the desired phenotype, applies the introduction *jun*B homzygous mutant ES cells into wild-type tetraploid blastocysts as described previously (Wang et al., 1997). This technique is able to rescue the early embryonic lethality of *jun*B mutant embryos and to allow for development of *jun*B mutant embryos up to mid-gestation (Schorpp-Kistner et al., 1999; our own results). Fetal liver cell suspensions containing hematopoietic stem cells are prepared from such E12.5 tetraploid rescued *jun*B mutant fetuses and are used to reconstitute lethally irradiated wild-type recipient mice according to standard procedures. In experiments of the invention we could demonstrate that *jun*B mutant fetal liver cells indeed reconstitute the hematopoietic system of lethally irradiated syngenic recipients.

Mice obtained according to these methods, are useful for the following purposes:

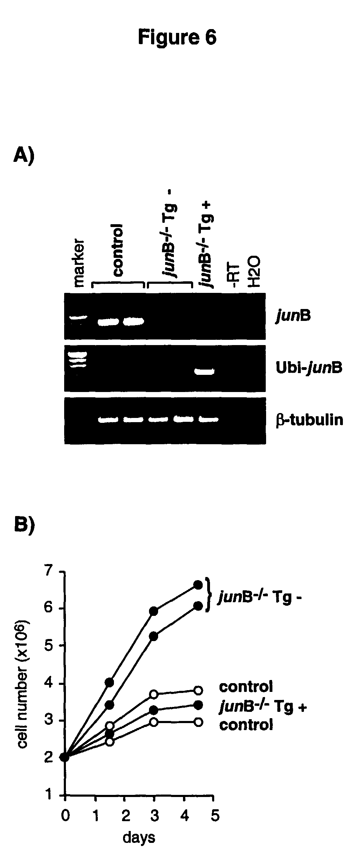

Firstly, mice lacking JunB expression in the myeloid lineage can be used as a source for establishing JunB deficient myeloid cell lines as described in Example 6. In the experiments of the invention, myeloid cell lines lacking JunB expression were successfully derived from bone marrow of *jun*B^{-/-}Ubi-*jun*B mice (Fig. 6). Alternatively, such cell lines can also be obtained from any mouse lacking JunB expression in the myeloid lineage.

Thus, the present invention relates to myeloid cells lacking JunB expression.

These cells are suitable tools for (a) the identification of drugs that inhibit the proliferation of myeloid cells and are thus useful for the treatment of CML, in particular during blast crisis, (b) the search for JunB target genes involved in the control of myeloid cell proliferation and differentiation, and (c) the screening for genetic events involved in the transformation of myeloid cells.

An example for conducting screening assays based on this principle is the following.

The JunB deficient myeloid cell lines can be used:
(a) to identify drugs or components that inhibit cell proliferation and induce terminal differentiation.
(b) to identify JunB target genes involved in myeloid cell proliferation and differentiation: For this purpose, artificial controls can be generated by re-introducing *jun*B in the JunB deficient myeloid cell lines. The cells are stably transfected or infected by conventional techniques with a recombinant DNA molecule containing the full-length mouse *jun*B gene fused in frame to any kind of inducible system such as the hormone-binding domain of the human estrogen receptor (ER) or the human progesterone receptor (PR). The principle of these inducible systems is that, although the chimaeric proteins are constitutively expressed, it is in an inactive state in the absence of the appropriate ligand such as exogenous β-estradiol for the estrogen receptor (Picard, 1994). These pseudo-control cells and *jun*B^{-/-}Ubi-*jun*B cells can be subject to analysis for identifying JunB target genes using methods known in the art to determine differential gene and protein expressions (e.g., differential display, RT-PCR analysis, DNA microarray technology, subtractive hybridization or proteome analysis).
(c) to identify genetic events associated with blastic transformation. Standard techniques for analyzing gene rearrangements and chromosome translocations can be used to search for common transformation events observed in a statistical number of independent JunB deficient myeloid cell lines.

Mice lacking *jun*B expression in the myeloid lineage can be used as an animal model to confirm that a candidate compound identified in a screening assay (a) to the anti-proliferative effect. The compound will be administered to the mouse and the effect of the compound will be monitored by determining its potential therapeutic effect, e.g. by measuring the absolute number of neutrophilic granulocytes in the peripheral blood. Compounds which reduce the number of these cells are candidates as drugs for the treatment of human CML, in particular during the chronic phase.

Mice lacking *jun*B expression in the myeloid lineage that are in blast crisis, can also be used as a source for appropriate material (tissue, cells, DNA, RNA, protein) which is analyzed by appropriate methods known in the art to confirm the *in vivo* manifestation of events identified in (c).

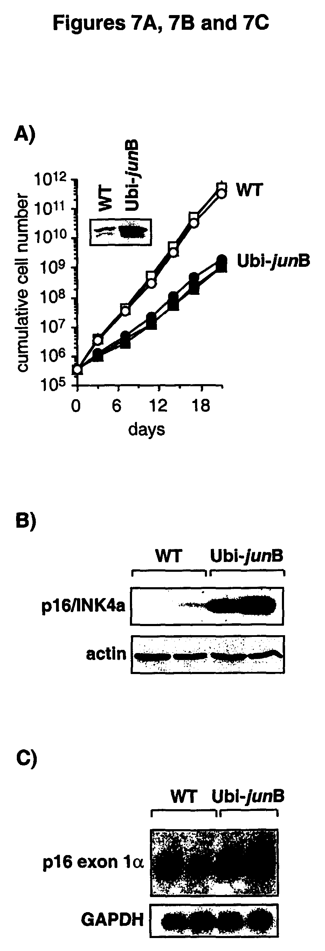

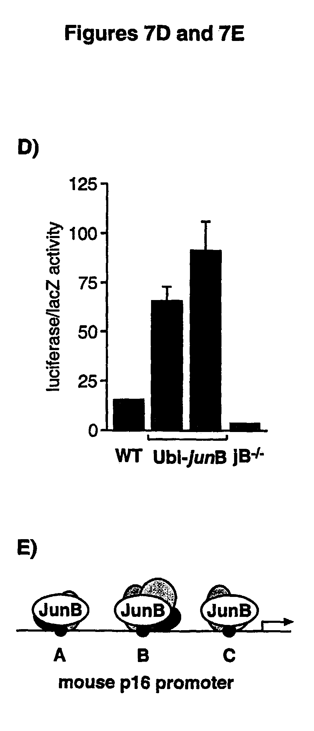

Furthermore, in the experiments of the present invention, the role of JunB in controlling cell proliferation was analyzed *in vitro* using fibroblasts derived from Ubi-*jun*B transgenic mice (Fig. 7). Spontaneously immortalized 3T3-fibroblasts expressing high JunB levels have a pronounced proliferation defect due to an extension of the G1-phase of the cell cycle. These fibroblasts have elevated levels of the cyclin-dependent kinase inhibitor p16/INK4a resulting in a complete inhibition of the cyclinD1/CDK4-6 kinase activity and reduced pRb phosphorylation. Finally, three JunB/AP-1 binding sites were characterized in the mouse p16 promoter through which JunB transcriptionally regulates p16 expression. These results identify JunB as a negative regulator of cell proliferation and provide a molecular link between growth factor signaling and the regulation of cell cycle progression by cyclin-dependent kinase inhibitors.

These findings combined with the *in vivo* results described above, allow to consider JunB as a potential tumor suppressor gene that could operate *in vivo* to cause growth arrest of cells during terminal differentiation processes, e.g. the growth arrest of myeloid cells during terminal differentiation.

Defining the role of JunB in cell cycle control and its mechanism of action provides, inter alia, the basis for methods for identifying drugs that enhance its tumor suppressor function either directly or indirectly. Examples for such drugs are substances increasing the activity of JunB target genes, e.g. p16 a negative regulator of the cell-cycle, or inhibiting the activity of downstream components of the JunB-mediated signal transduction pathway, e.g. the cyclinD/CDK4-6 complex, a positive regulator of the cell-cycle.

Regulatory sequences of JunB target genes as well as newly discovered JunB target genes can be used to monitor the effect of drugs suppressing the inhibitory effect of JunB on cell proliferation.

An example for conducting screening assays based on this principle is the following:

Suitable test cells are fibroblasts expressing high levels of JunB protein, e.g. fibroblasts derived from Ubi-*jun*B transgenic mice as described in Example 7. The cells are stably transfected, by conventional techniques, with a recombinant DNA molecule containing the regulatory regions of a JunB target gene, e.g. p16, fused to a reporter gene, according to known methods. Any reporter gene may be used whose expression product is detectable by measuring a signal proportional to its concentration and which can be detected and quantified. Preferably, a reporter gene is employed whose expression can be measured with high sensitivity in an automated assay such as the luciferase gene, the Green Fluorescent Protein (GFP) gene and the LacZ gene. The cells are grown in suitable medium in presence or absence of the substance to be tested and are assayed for their effects on (1) proliferation of the cells and (2) reporter gene activity. Substances which exhibit the desired effect will increase proliferation of the test cells and down-regulate reporter gene activity.

In a second step, identified substances can be tested on bone marrow-derived primary myeloid cells for their effects on proliferation. Substances which exhibit the desired effect will increase the proliferation rate of myeloid cells and can be considered in view to the application as human drugs to burst myeloid cell proliferation after, for example, chemotherapy or radiotherapy.

In a further aspect, the invention relates to pharmaceutical compositions comprising, as an active ingredient, a substance which enhances the activity of JunB as a negative regulator of proliferation of tumor cells. Substances that enhance JunB activity are therefore useful as antitumor agents. They may act according to various mechanisms, e.g. by increasing JunB expression, JunB stability, JunB DNA binding and/or transactivation activity.

In a further aspect, the invention relates to pharmaceutical compositions comprising, as an active ingredient, a substance which inhibits JunB activity as a negative regulator of cell proliferation in normal cells. Substances that inhibit JunB activity are therefore useful as agents promoting the regeneration of cells such as myeloid cells in situations of impaired hematopoiesis, e.g. after chemotherapy or radiotherapy. They may act according to various mechanisms e.g. by reducing JunB expression, JunB stability, JunB DNA binding and/or transactivation activity.

### Brief description of the Figures

Fig. 1
   Breeding scheme to obtain *jun*B^{-/-} mice carrying the Ubi*-jun*B transgene
Fig. 2
   A) RT-PCR analysis of expression levels for the endogenous *jun*B gene and the Ubi-*jun*B transgene in various FACS-sorted haemopoietic lineages.
   B) Western-blot analysis of JunB protein levels in FACS-sorted B cells and myeloid cells.
Fig. 3
   FACS analysis of the myeloid compartment from spleen and bone marrow of control and *jun*B^{-/-}Ubi-*jun*B mice.
Fig. 4
   A) FACS analysis of the bone marrow myeloid compartment of recipient mice transplanted with control and *jun*B-/-Ubi-*jun*B bone marrow cells.
   B) Genotyping of the recipient mice
Fig. 5
   Cell cycle profile of spleenic myeloid population from control and *jun*B-/-Ubi-*jun*B mice.
Fig. 6
   A) RT-PCR analysis of expression levels for the endogenous *jun*B gene and the Ubi-*jun*B transgene in myeloid cell lines derived from control and *jun*B^{-/-}Ubi-*jun*B mice.
   B) Proliferation rates of control and JunB deficient myeloid cell lines.
Fig. 7
   A) Proliferation rates of wild-type and Ubi-*jun*B transgenic 3T3-fibroblasts.
   B) Western blot analysis of p16 protein levels in wild-type and Ubi-*jun*B transgenic 3T3-fibroblasts.
   C) Northern blot analysis of p16 mRNA levels in wild-type and Ubi-*jun*B transgenic 3T3-fibroblasts.
   D) p16 transcriptional activity in wild-type, Ubi-*jun*B transgenic and *jun*B^{-/-} 3T3-fibroblasts.
   E) JunB/AP-1 binding sites identified in the murine p16 promoter.

### Example 1

### Generation of junB^{-/-}Ubi-junB mice

*jun*B^{-/-}Ubi-*jun*B mice were obtained according to the method described by Schorpp-Kistner et al. (1999). In brief, Ubi-*jun*B transgenic mice were crossed to *jun*B mutant heterozygous mice and the transgenic *jun*B mutant heterozygous progenies were intercrossed to generate mice homozygous for the inactivated *jun*B allele that carry the Ubi-*jun*B transgene. The approach used is schematically outlined in Fig. 1. Such *jun*B^{-/-}Ubi-*jun*B mice were obtained in the expected Mendelian ratio, which is given in Table 1.

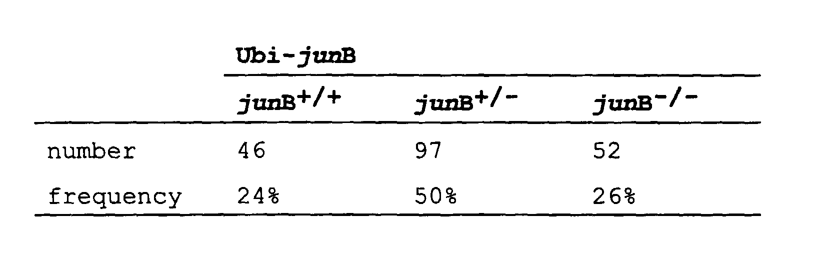

### Example 2

### Analysis of JunB expression in various hematopoetic lineages

To examine JunB expression in the hematopoietic system of *jun*B^{-/-}Ubi-*jun*B mice, various hematopoietic lineages were FACS-sorted by known methods (Coligan et al., 1992) according to their cell surface marker constellations. mRNA and protein preparations of these cell populations were obtained according to standard methods (Sambrook et al., 1989). RT-PCR and protein analysis revealed (Fig. 2) that only myeloid cells (M: Gr-1+/Mac-1+) from *jun*B^{-/-}Ubi-*jun*B mice were lacking JunB expression, while JunB expression was detected in B lymphocytes (B: CD19+/Ig-M+), T helper lymphocytes (T: CD4+/CD8-) and erythroid cells (E: TER-119+). This expression profile indicates that *jun*B^{-/-}Ubi-*jun*B mice display a cell type-specific loss of JunB expression in the myeloid lineage.

### Example 3

### Analysis of the phenotype of junB^{-/-}Ubi-junB mice

All *jun*B^{-/-}Ubi-*jun*B mice obtained in Example 1 developed a myeloproliferative disease with complete penetrance by 11 months of age. Hematological analysis of peripheral blood by measuring absolute blood cell counts and evaluating stained blood smears revealed a 10-fold increase in leukocyte numbers in *jun*B^{-/-}Ubi-*jun*B mice at 6 to 11 months of age. The increase was largely due to elevated numbers of neutrophilic granulocytes. The numbers of other hematopoietic lineages such as erythrocytes, lymphocytes, monocytes and eosinphilic granulocytes were comparable to wild-type controls. However, reduced hemoglobin concentrations indicated the presence of hypochromic anaemia. The results are given in Table 2 (n = number of mice analyzed for each genotype).

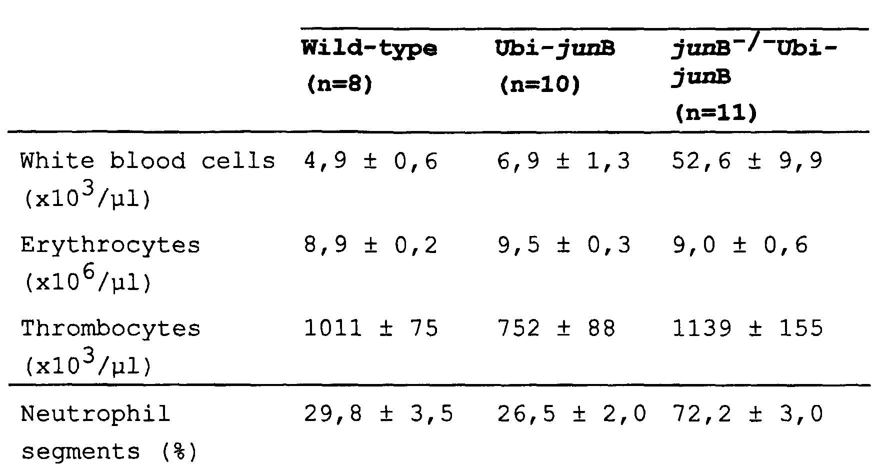

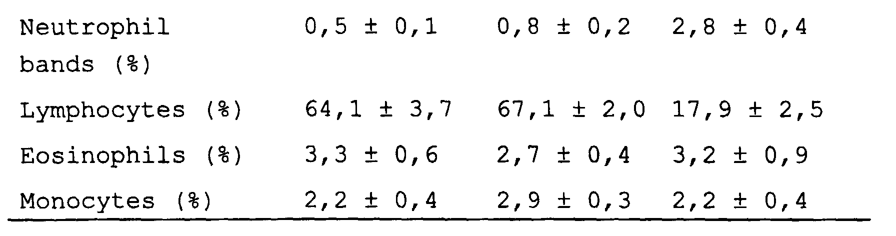

Gross anatomical analysis of diseased *jun*B^{-/-}Ubi-*jun*B mice consistently revealed a massive enlargement of the spleen and lymph nodes. Histological examination of spleen sections showed pronounced myeloid hyperplasia indicated by infiltration of the red pulp by myeloid cells of various maturation stages. Histochemical stains for chloroacetate esterase indicated the presence of numerous mature neutrophilic granulocytes.

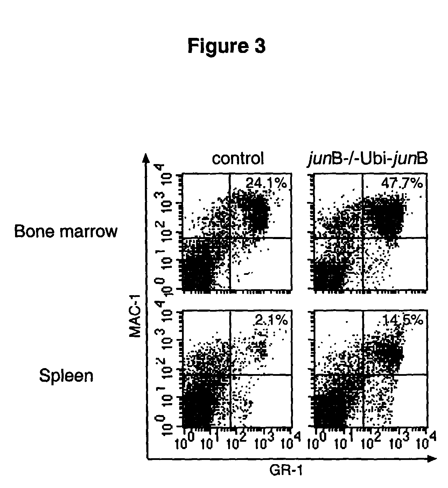

Stained cytospin preparations of spleen cells confirmed the presence of numerous myeloid cells, mainly mature granulocytes. The bone marrow of *jun*B^{-/-}Ubi-*jun*B mice showed increased cellularity and myeloid hyperplasia as determined by the investigation of cytospin preparations and histological sections of bone marrow. Histological analysis of *jun*B^{-/-}Ubi-*jun*B livers showed infiltration of the portal tracts by mature and immature cells of the granulocyte lineage. FACS analysis of single cell suspensions of bone marrow and spleen cells using antibodies directed to cell type specific surface marker showed a drastic expansion of the myeloid compartment with consecutive reduction of the lymphocyte compartments (Fig. 3).

In 10 to 20% of *jun*B^{-/-}Ubi-*jun*B mice disease progression to blast crisis was observed as indicated by the infiltration of haemopoietic or non-hematopoietic organs such as heart, lung, kidney and skin by immature blastic cells.

### Example 4

### Bone marrow transplantation experiment to demonstrate that the CML-like disorder is a cell autonomous defect

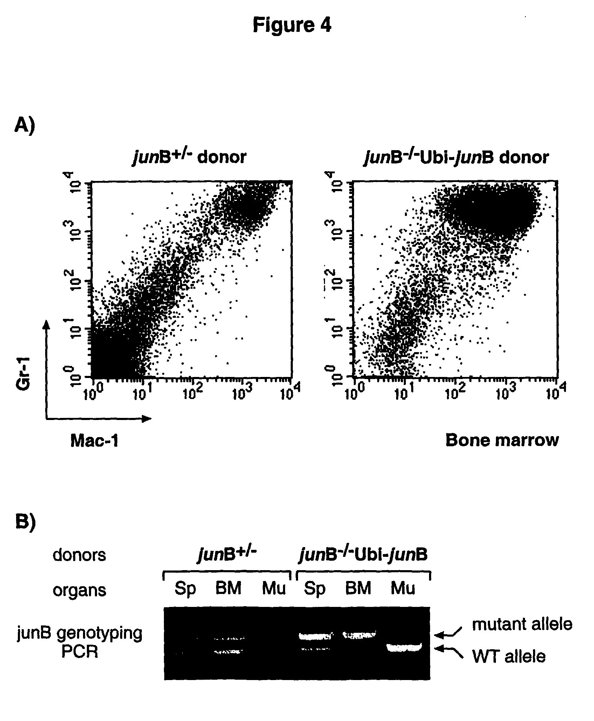

Myelopoiesis is controlled by cytokines such as IL-3, IL-6, GM-CSF and G-GSF, which are able to stimulate myeloid proliferation and differentiation *in vitro* and *in vivo*. To test whether the myeloproliferative disease of *jun*B^{-/-}Ubi-*jun*B mice is due to a cell autonomous defect, bone marrow cells were transplanted into lethally irradiated wild-type recipients as described (Keller and Wagner, 1989). Recipients were followed for the development of the myeloproliferative disease by stained blood smears. Within a follow-up period of 8 months, 85% of recipients developed a disease characterized by elevated neutrophilic granulocyte counts in peripheral blood smears and expansion of the myeloid compartment in spleen and bone marrow as measured by FACS analysis (Fig. 4A). PCR-based analysis of DNA extracted from bone marrow and spleen cells of recipients indicated complete reconstitution of the recipients by *jun*B^{-/-}Ubi-*jun*B bone marrow cells (Fig. 4B).

### Example 5

### In vivo analysis of junB^{-/-}Ubi-junB myeloid cell proliferation

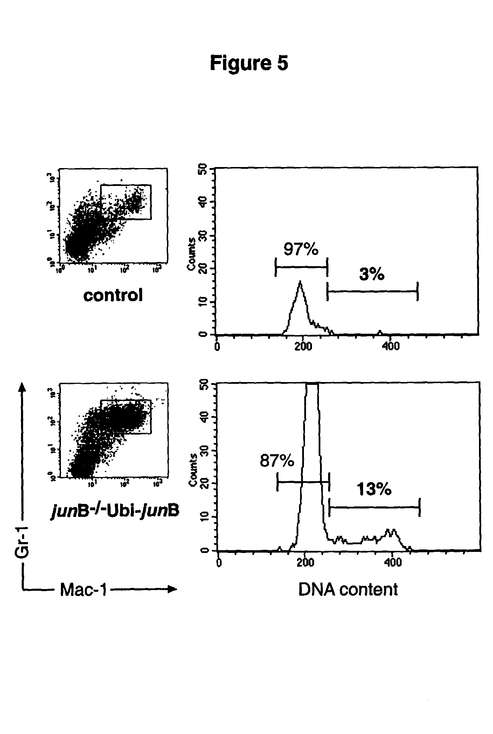

To study whether the expansion of the myeloid compartment was due to increased proliferation of precursor cells within the lineage, the DNA content of myeloid spleen cells (Gr-1+/Mac-1+) was analyzed by FACS using a similar method as in Example 2. In *jun*B^{-/-}Ubi-*jun*B mice, 13 % of the myeloid cell population was found in S and G₂/M phase of the cell cycle, compared to 3 % in control mice, showing increased number of cycling cells in the myeloid population of *jun*B^{-/-}Ubi-*jun*B mice (Fig. 5). This demonstrates that JunB deficient myeloid cells display increased proliferation *in vivo.*

### Example 6

### Derivation of myeloid cell lines lacking JunB expression

Immortalized myeloid cell lines lacking junB expression were generated from *jun*B^{-/-}Ubi-*jun*B mice. Bone marrow cells were isolated from the long bones of the mice and grown in methylcellulose medium containing a combination of growth factors and cytokines supporting proliferation and differentiation along the granulocytic lineage (e.g., GM-CSF, G-CSF, SCF, IL-3 and IL-6) as described in Liu et al. (1996). After 7 days, single differentiated colonies were picked from the methylcellulose and grown in liquid culture containing the same cocktail of growth factors and cytokines. After 2-3 weeks, wild-type derived colonies stopped proliferating and terminally differentiated. In contrast, colonies derived from *jun*B^{-/-}Ubi-*jun*B mice that lack JunB expression kept proliferating and were immortalized (Fig. 6).

### Example 7

### JunB suppresses 3T3-fibroblasts proliferation by transcriptionally activating expression of the cyclin-dependent kinase inhibitor p16/INK4a.

To determine the effect of high JunB expression levels on cell proliferation, mouse embryonic fibroblasts were derived from E12.5 Ubi-*jun*B transgenic embryos (Schorpp et al., 1996) and established according to the 3T3-protocol (Todaro and Green, 1963). All established Ubi-*jun*B transgenic 3T3-fibroblast lines proliferated slowly (Fig. 7A) with a doubling time of 43 ± 2.5 hours, compared to 19.4 ± 1.3 hours in the wild-type counterparts, and saturation densities of only 50% of the wild-type cells.

To identify the mechanism through which JunB impairs fibroblasts proliferation, the expression levels of members of the p21 and p16 families of cyclin-dependent kinase inhibitors (CKIs) were investigated. CKIs are known to negatively regulate cell cycle progression through binding and inhibition of cyclin/CDK complexes (Sherr and Roberts, 1995). Only the level of the CKI p16/INK4a was found to be dramatically elevated at both the protein (Fig. 7B) and the mRNA (Fig. 7C) levels in all Ubi-*jun*B transgenic 3T3-fibroblasts tested.

To study whether JunB can transactivate the p16 promoter, a 1.2 kb 5'-fragment of the mouse p16 promoter was cloned into a luciferase reporter construct (p16-Luc) and transient transfection experiments were performed with wild-type, Ubi-*jun*B transgenic and *jun*B^{-/-} 3T3-fibroblasts (Fig. 7D). Wild-type fibroblasts showed a moderate level of p16-Luc activity consistent with the low level of p16 mRNA and protein expressed. In contrast, Ubi-*jun*B transgenic cells exhibited 3 to 5 times more p16 transcriptional activity whereas in *jun*B^{-/-} cells only half of the activity was detected compared to wild-type cells.

Finaly, we identified three JunB/AP-1 binding sites in the murine p16 promoter (depicted in Fig. 7E) and demonstrated that they are critical for both basal and JunB-mediated p16 expression since their mutation results in both decreased p16 transactivation in wild-type cells and complete inhibition of JunB-mediated p16 transactivation.

### REFERENCES

Angel P and Karin M (1991). Biochim. Biophys. Acta 1072, 129-157
Araki K, Araki M, Mijazaki J, Vassalli P (1995) Proc. Natl. Acad. Sci. 92: 160
Bonham L, MacKenzie K, Wood S, Rowe PB, Symonds G (1992) Oncogene 7:2219-2229
Brown D, Kogan S, Lagasse E, Weissmann I, Alcalay M, Pelicci PG, Atwater S, Bishop JM (1997) Proc. Natl. Acad. Sci. USA 94:2551-2556
Capecchi MR (1989) Science 244: 1288-1292
Cesana A, Hoxie JA, Lange B. Nowell PC, Bishop J, Santoli D (1992) Oncogene 7:827-836
Chang JM, Metcalf D, Gonda TJ, Johnson GR (1989a) J. Clin. Invest. 84:1488
Chang JM, Mecalf D, Lang RA, Gonda TJ, Johnson GR (1989b) Blood 73:1487-1497
Chiu R, Angel P, and Karin M (1989) Cell 59: 979-986
Coligan JE, Kruisbeek AM, Margulies DH, Shevach EM and Strober W (1992) Current Protocols in Immunology, NIH press (second edition)
Daley GQ, Van Etten RA, Baltimore D (1990) Science 247:824-830
Deininger MWN and Goldman JM (1998) Curr. Opin. Hematol. 5:302-308
Deng T and Karin M (1993) Genes Dev. 7: 479-490
Dymecki SM (1996). Proc. Natl. Acad. Sci. USA 93:6191-6196
Elefanty GA, Hariharan IK, Cory S (1990) EMBO J. 9:1069-1078
Grisolano JL, Wesselschmidt RL, Pelicci PG, Ley TJ (1997) Blood 89:376-387
Hawley RG, Fong AZC, Burns BF, Hawley TS (1992) J. Exp. Med. 176:1149-1163
Heisterkamp N, Jenster G, ten Hoeve J, Zovich D, Pattengale PK, Groffen J (1990) Nature 344:251-253
Helgason CD, Damen JE, Rosten P, Grewal R, Sorensen P, Chappel SM, Borowsky A, Jirik F,
Holtschke T, Löhler J, Kanno Y, Fehr T, Giese N, Rosenbauer F, Lou J, Knobeloch K-P, Gabriele L, Waring JF, Bachmann MF, Zinkernagel RM, Morse III HC, Ozato K, Horak I (1996) Cell 87:307-317
Kelliher MA, McLaughlin J, Witte ON, Rosenberg N (1990) Proc. Natl. Acad. Sci. USA 87:6649-6653
Krystal G, Humphries RK (1998) Genes Dev. 12:1610-1620
Johnson GR, Gonza TJ, Metcalf D, Hariharan IK, Cory S (1989) EMBO J 8:441-448
Keller G and Wagner EF (1989) Genes Dev. 3:827-837
Lagasse E and Weissmann I (1994) J. Exp. Med. 1047-1052
Largaespada DA, Brannan CI, Jenkins NA, Copeland NG (1996) Nat. Gen. 12:137-143
Li B, Tournier C, Davis RJ, Flavell RA (1999) EMBO J 18: 420-432
Liebermann DA, Hoffmann-Liebermann B (1994) Curr. Opin. Hemato. 1: 24-32
Liu F, Wu HY, Wesselschmidt R, Kornaga T and Link DC (1996) Immunity 5: 491-501
Lord KA, Hoffmann-Liebermann B, Liebermann, DA (1990) Oncogene 5: 387-396
Lord KA, Abdollahi A, Hoffmann-Liebermann B, Liebermann DA (1993) Mol. Cell. Biol. 13:841-851
MacKenzie KL, Dolnikov A, Millington M, Shounan Y, Symonds G (1999) Blood 93:2043-2056
Mollinedo F, Vaquerizo MJ, Naranjo JR (1991) Biochem. J. 273:477-479
Mollinedo F and Naranjo JR (1991) Eur. J. Biochem. 200: 483-486
Picard, D (1994 ) Curr. Opin. Biotechnol. 5: 511-515
Sambrook J, Fritsch EF and Maniatis T (1989) Molecular Cloning, Cold Spring Harbor Laboratory Press (second edition)
Sawyers CL, Gishizky ML, Quan S, Golde DW, Witte ON (1992) Blood 79:2089-2098
Sawyers CL (1999) New Engl. J. Med. 340:1330-1340
Schlingensiepen KH, Schlingensiepen R, Kunst M, Klinger I, Gerdes W, Seifert W, Brysch W (1993) Dev. Genet. 14: 305-312
Schorpp-Kistner M, Wang Z-Q, Angel P, and Wagner EF (1999) EMBO J 18:934-948
Schorpp M, Jäger R, Schellander K, Schenkel J, Wagner EF, Weiher H, Angel P (1996) Nucleic. Acids. Res. 24: 1787-1788
Schwaller J, Frantsve J, Aster J, Williams IR, Tomasson MH, Ross TS, Peeters P, Van Rompaey L, Van Etten RA, Ilaria R Jr, Marynen P, Gilliland DG (1998) EMBO J 17:5321-5333
Sherr CJ and Roberts JM (1995). Genes Dev. 9, 1149-1163
Shounan Y, MacKenzie K, Dolnikov A, Miller M, Symonds G (1997) Leukemia 11:1641-1649
Tenen DG, Hromas R, Licht JD, Zhang DE (1997) Blood 90:489-519
Todaro GJ and Green H (1963). J. Cell Biol. 17, 299-313
Wang Z-Q, Kiefer F, Urbáneck P, Wagner EF (1997) Mech. Dev. 62:137-145
Wong PMC, Chung S-W, Dunbar CE, Bodine DM, Ruscetti S, Nienhuis AW (1989) Mol. Cell. Biol.:9:798-808
Yan X-Q, Brady G, Iscove NN (1994) Oncogene 9:163-173

## Claims

1. The use of a transgenic animal lacking JunB expression in the myeloid lineage as a model for human chronic myeloid leukemia.

2. The use of claim 1, wherein said animal is a mouse.

3. The use of a transgenic animal defined in claim 1 or 2, said animal being obtainable by transgene complementation of a *jun*B knock-out animal and application of a *jun*B transgene in which *jun*B is under the control of a strong and constitutive promoter that leads to expression during early embryonic development.

4. The use of claim 3, wherein said promotor is the ubiquitin C promotor.

5. The use of a transgenic animal defined in claim 1 or 2, said animal being obtainable by conditional inactivation of *jun*B in the myeloid lineage.

6. The use of a transgenic animal defined in claim 1 or 2, said animal being obtainable by introduction of *jun*B homzygous mutant ES cells into wild-type tetraploid blastocysts

7. JunB deficient myeloid cells obtainable from a transgenic animal defined in claim 1 or 2.

8. The use of JunB deficient myeloid cells for the identification of drugs that inhibit the proliferation of myeloid cells.

9. A pharmaceutical composition comprising, as an active ingredient, a substance which enhances the activity of JunB as a negative regulator of proliferation of tumor cells.
